# EUROPEAN PATENT APPLICATION

(11) **EP 4 811 176 A1**
(43) Date of publication of application: **23.09.2026**
(21) Application number: 24890208.2
(22) Date of filing: 07.08.2024
(51) Int. Cl.: G06F 18/10, A61B 5/369

(54) **METHOD, APPARATUS AND PROCESSOR FOR REALIZING METHAMPHETAMINE ADDICTION IDENTIFICATION AND CRAVING DETERMINATION ON BASIS OF MACHINE LEARNING, AND COMPUTER-READABLE STORAGE MEDIUM THEREFOR**

(30) Priority: 16.11.2023 CN 202311529756
(71) Applicant: Shanghai Mental Health Center (Shanghai Psychological Counselling Training Center), Shanghai 200030 (CN)
(72) Inventor: YUAN, Tifei, Shanghai 200030 (CN); ZHAO, Di, Shanghai 200030 (CN); WU, Wei, Shanghai 200030 (CN); TIAN, Weiwen, Shanghai 200030 (CN)
(74) Representative: Dummett Copp LLP
(86) International application number: PCT/CN2024/110274
(87) International publication number: WO 2025/102844

(57) **Abstract**

The present invention relates to a method for realizing methamphetamine addiction identification and craving determination based on machine learning, wherein the method comprises the following steps: collecting and preprocessing high-density scalp resting-state electroencephalogram data of a subject to obtain an EEG time series; performing source localization on the acquired time series data based on a standard head model; performing electroencephalogram functional connectivity analysis using a predefined brain template; detecting temporal correlations or phase synchronization between multiple brain regions using a functional connectivity network; and performing feature discrimination of the subject's true craving performance by establishing and validating a machine learning model. The present invention also relates to a corresponding apparatus, processor and storage medium therefor. By adopting the method, apparatus, processor and storage medium for realizing methamphetamine addiction identification and craving determination based on machine learning of the present invention, it is expected to accurately identify methamphetamine addicts and their craving states, help evaluate the treatment effect of methamphetamine addicts, and reduce the relapse rate.

## Description

### CROSS-REFERENCE TO RELATED APPLICATIONS

This application claims the priority of the Chinese Invention Patent Application No. 202311529756.0 filed on November 16, 2023, the contents of which are incorporated into this application by reference.

### TECHNICAL FIELD

The present invention relates to the field of biomedical technology, in particular to the field of neuroscience technology, and specifically refers to a method, apparatus, processor and computer-readable storage medium for realizing methamphetamine addiction identification and craving determination based on machine learning.

### BACKGROUND ART

"Methamphetamine" addicts exhibit behavioral characteristics such as persistent psychological dependence on drugs (high craving) and abnormal impulsivity, all of which mediate high-risk behaviors such as relapse and violence, imposing a heavy burden on family economies and social stability. Among them, craving is the direct cause of relapse in addicts. Identifying electroencephalogram (EEG) markers of craving in methamphetamine addicts can serve as objective markers of relapse during the withdrawal period, which is crucial for assisting doctors in the diagnosis, monitoring, treatment and prognosis of methamphetamine addicts.Scalp electroencephalography (EEG) has the advantages of high temporal resolution, low cost and easy data acquisition, providing a feasible approach for translating biomarkers of craving in methamphetamine addicts. However, traditional 32- or 64-channel scalp EEG performs poorly in source localization analysis, where the influence of volume conduction can significantly alter scalp EEG signals. Notably, the use of high-density EEG (128 channels or higher) can largely mitigate the impact of volume conduction. High-density EEG systems can provide source reconstruction of various brain regions and global activities, through which brain activity can be reconstructed by applying precise source imaging.

Existing technologies:
1. Hypothesis-driven approach: Researchers compare different groups (e.g., a group of methamphetamine addicts and a group of healthy controls) to explore the clinical relevance of specific neural signals based on a priori hypotheses. For example, previous studies have shown that increased slow-wave activity (δ and θ) and increased β-band synchronization in the medial prefrontal cortex (MPFC) are associated with enhanced cue-induced craving in methamphetamine abusers. In this approach, prior EEG evidence is used to determine the neural indicators to be measured, the population to be tested, and the analytical methods to be performed. Using this approach, although multiple brain regions have been identified as associated with methamphetamine craving and can distinguish healthy controls from methamphetamine addicts, these associations and statistically significant differences have not produced robust biomarkers for methamphetamine addicts. Hypothesis-driven approaches attempt to elucidate the variance or differences between multiple variables but have limited applicability to new individuals (1,2).
2. Data-driven machine learning approach: This method uses various neurobiological features for classification of methamphetamine addicts and prediction of craving. Previous studies have proposed using the mean and standard deviation of absolute EEG power and skin conductance response to classify methamphetamine addicts and healthy controls (3,4), but relatively low-density EEG poses challenges in effectively modeling the topological features of input connections. To date, high-density EEG biomarkers have not been successfully used to predict methamphetamine addiction and identify methamphetamine addicts at the individual level in a reliable manner.

### SUMMARY OF THE INVENTION

The object of the present invention is to overcome the shortcomings of the above-mentioned prior art and provide a method, apparatus, processor and computer-readable storage medium for realizing methamphetamine addiction identification and craving determination based on machine learning.

To achieve the above object, the method, apparatus, processor and computer-readable storage medium for realizing methamphetamine addiction identification and craving determination based on machine learning of the present invention are as follows:
The method for realizing methamphetamine addiction identification and craving determination based on machine learning comprises the following steps:
(1) Collecting high-density scalp resting-state EEG data from subjects;
(2) Preprocessing the collected data to remove noise data and artifacts, obtaining preprocessed EEG time series;
(3) Performing brain region source localization processing on the acquired time series data using a predefined brain template based on a standard head model;
(4) Detecting temporal correlations or phase synchronization between multiple brain regions using a functional connectivity network;
(5) Performing feature discrimination of the subjects' true craving performance by establishing and validating a machine learning model.

Preferably, step (1) is specifically:
Adjusting all 128 scalp channels of the high-density EEG cap to maintain impedance <50kΩ, with a sampling rate of 500Hz, and performing offline filtering on the collected resting-state EEG data generated by methamphetamine-induced cues using a 0.01-100Hz bandpass filter or a 50Hz notch filter.

Preferably, step (2) specifically comprises the following steps:
(2.1) Performing offline preprocessing on the collected high-density EEG data, downsampling the time series to 250Hz, removing 50Hz power frequency noise through a notch filter, and performing bandpass filtering between 1Hz and 100Hz through a zero-phase finite impulse response filter;
(2.2) Removing bad channels including bridged channels and noisy channels and performing spherical interpolation, excluding and excising paroxysmal segments; decomposing independent components using independent component analysis to remove artifacts such as blinks, eye movements, heartbeats and electromyography;
(2.3) Detecting and removing bad components through dimensionality reduction using principal component analysis, and determining the minimum number of principal components accounting for more than 99.9% of the total variance as the dimension, thereby obtaining preprocessed EEG time series;
(2.4) Filtering the sensor-level preprocessed time series into five typical frequency bands: delta (1-3Hz), theta (4-7Hz), alpha (8-12Hz), beta (13-30Hz) and gamma (31-50Hz) through a custom pipeline and verification by EEG experts, thereby completing data preprocessing. Preferably, step (3) specifically comprises the following steps:
(3.1) Calculating a three-layer symmetric boundary element model of the head using OpenMEEG, then selecting rotating dipoles at 3003 vertices on the cortical surface, estimating an imaging kernel using a minimum norm estimation method with depth weighting and regularization, wherein the imaging kernel maps scalp electrode EEG to cortical source-level current source density, and then reducing the current density time series of each vertex in three spatial dimensions from three orthogonal axes to one dimension through principal component analysis;
(3.2) Generating a time series representing the main variation patterns of all vertices in the same region of interest through principal component analysis, extracting the dominant signal of the time series using singular value decomposition, and regarding the first singular vector as the signal at the region of interest level;
(3.3) The 31 regions of interest are independently divided according to the functional connectivity of resting-state functional magnetic resonance imaging, including:
   - VIS (Visual Network): Bilateral primary visual areas (V1);
   - Somatosensory Network: Bilateral primary somatosensory cortex (S1);
   - DAN (Dorsal Attention Network): Inferior frontal junction (IFJ), intraparietal sulcus (IPS), frontal eye field (FEF) and supplementary eye field (SEF);
   - DMN (Default Mode Network): Posterior cingulate cortex (PCC), medial prefrontal cortex (MPFC) and angular gyrus (ANG);
   - FPN (Frontoparietal Control Network): Posterior middle frontal gyrus (PMFG), inferior parietal lobule (IPL), orbital gyrus (ORB) and middle temporal gyrus (MTG);
   - VAN (Ventral Attention Network): Anterior middle frontal gyrus (AMFG), insular gyrus (INS), dorsal anterior cingulate cortex (DACC) and supramarginal gyrus (SUP).

Preferably, step (4) is specifically:

Calculating the imaginary coherence connectivity of 465 unique ROI pairs for each of the five frequency bands and each of the two resting eye conditions to obtain interactions between brain regions of interest at the cortical level. Let *S_{i,j,t}*(*f*) denote the cross-spectral density of *x_{_i}*(*t*) and *x_{_j}*(*t*) at time point or trial t, where *S_{i,j,t}*(*f*) is expressed as: ${S}_{ij , t} \left(f\right) = \left〈{x}_{i , t}\left(f\right){{x}_{j , t}}^{*}\left(f\right)\right〉 ,$ where * denotes complex conjugate and < > denotes expected value;

Let *S_{_i,j}*(*f*) denote the cross-spectrum, and imaginary coherence is defined as: ${iCoh}_{ij} \left(f\right) = Imag \left({Coh}_{ij}\left(f\right)\right) = \frac{Imag \left({S}_{ij}\left(f\right)\right)}{\sqrt{{S}_{ii} \left(f\right) ⋅ {S}_{jj} \left(f\right)}} .$

Preferably, step (5) specifically comprises the following steps:
(5.1) Performing group-level analysis on source-space FCNs and correcting the p-value of each pair of functional connections using false discovery rate adjustment;
(5.2) Using biomarker-based machine learning models for craving prediction in addicted individuals and identification of abnormal addicted individuals to assess the strength of the relationship between the two biomarkers;
(5.3) Constructing a sparse linear regression model for predicting craving scores from connectivity features using a Relevance Vector Machine (RVM) with a linear kernel for high-density EEG data, and establishing a sparse classification model to distinguish methamphetamine addicts from healthy individuals using an RVM with a linear kernel for high-density EEG data and a sigmoid function outer layer;
(5.4) Using the RVM machine learning model to penalize complex models by maximizing marginal likelihood with sparse priors, thereby automatically selecting relevant features for prediction;
(5.5) Craving prediction for methamphetamine addicts: Predicting craving scores in methamphetamine addicts based on brain functional connectivity in regions of interest using an RVM model with 10 repetitions of 10-fold cross-validation;
(5.6) Abnormality identification for methamphetamine addicts: Classifying methamphetamine addicts and healthy control individuals using an RVM model with 10 repetitions of 10-fold cross-validation based on high-density EEG functional connectivity estimates, wherein the model assigns label "1" to individuals with methamphetamine addiction and label "0" to healthy control individuals, and evaluating classification performance using classification accuracy, sensitivity, specificity and area under the receiver operating characteristic curve;
(5.7) Abnormality identification and craving prediction using sensor-space power: Using the power spectrum of each frequency band in a single region of interest space as input features for the machine learning model, and comparing the classification and prediction performance between cortical functional connectivity and frequency band power spectra.

The apparatus for realizing methamphetamine addiction identification and craving determination based on machine learning comprises:
A processor configured to execute computer-executable instructions;
A memory storing one or more computer-executable instructions, which when executed by the processor, implement the steps of the above-described method for realizing methamphetamine addiction identification and craving determination based on machine learning.

The processor for realizing methamphetamine addiction identification and craving determination based on machine learning is configured to execute computer-executable instructions, which when executed by the processor, implement the steps of the above-described method for realizing methamphetamine addiction identification and craving determination based on machine learning.

The computer-readable storage medium has a computer program stored thereon, which can be executed by a processor to implement the steps of the above-described method for realizing methamphetamine addiction identification and craving determination based on machine learning.

By adopting the method, apparatus, processor and computer-readable storage medium for realizing methamphetamine addiction identification and craving determination based on machine learning of the present invention, major innovations have been brought to the diagnosis and treatment of methamphetamine addiction by combining high-density scalp EEG data and analysis of neural connection networks, providing a new approach for individual-level treatment methods, which is expected to improve the treatment effect of methamphetamine addicts and reduce the relapse rate.

### BRIEF DESCRIPTION OF THE DRAWINGS

FIG. 1 is a schematic diagram of high-density EEG data collection and feature extraction according to the method for realizing methamphetamine addiction identification and craving determination based on machine learning of the present invention.

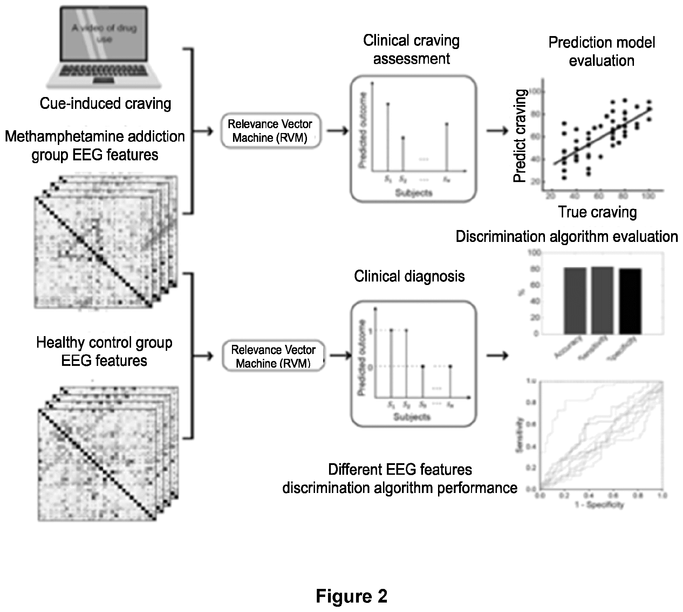

FIG. 2 is a schematic diagram of establishing and validating a machine learning model for the method for realizing methamphetamine addiction identification and craving determination based on machine learning of the present invention.

### DETAILED DESCRIPTION OF THE EMBODIMENTS

In order to more clearly describe the technical content of the present invention, further description is given below in conjunction with specific embodiments.

Before describing in detail embodiments according to the present invention, it should be noted that hereinafter, the terms "comprises", "includes" or any other variation are intended to cover non-exclusive inclusion, whereby a process, method, article or apparatus comprising a series of elements includes not only those elements but also other elements not explicitly listed, or elements inherent to such process, method, article or apparatus.

This technical solution specifically consists of the following components:
High-density EEG data acquisition and feature extraction:
(1) Recruiting methamphetamine addicts and healthy individuals;
(2) High-density scalp EEG data acquisition, preprocessing and source localization analysis;
(3) Obtaining the activity of 31 brain regions and EEG functional connectivity between 31 brain regions at rest;

Establishing and validating machine learning models to form software:
(4) Establishing an EEG feature machine learning model using Relevance Vector Machine to predict methamphetamine craving;
(5) Establishing an EEG feature machine learning model using logistic regression to identify methamphetamine addicts;
(6) Validating both models on additional datasets.

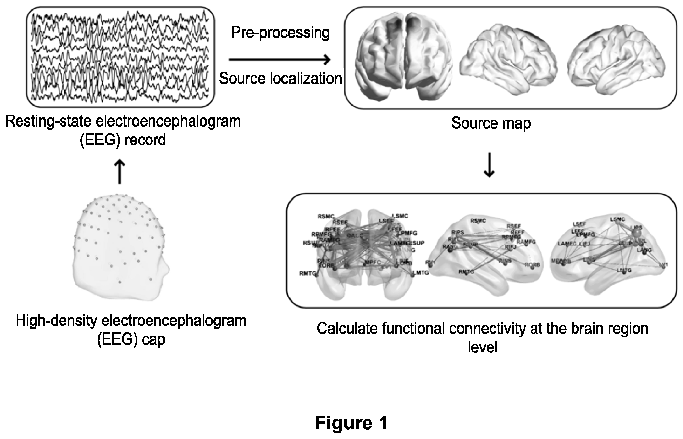

Referring to FIG. 1, the method for realizing methamphetamine addiction identification and craving determination based on machine learning comprises the following steps:
(1) Collecting high-density scalp resting-state EEG data from subjects;
(2) Preprocessing the collected data to remove noise data and artifacts, obtaining preprocessed EEG time series;
(3) Performing brain region source localization processing on the acquired time series data using a predefined brain template based on a standard head model;
(4) Detecting temporal correlations or phase synchronization between multiple brain regions using a functional connectivity network;
(5) Performing feature discrimination of the subjects' true craving performance by establishing and validating a machine learning model.

As a preferred embodiment of the present invention, step (1) is specifically:
Adjusting all 128 scalp channels of the high-density EEG cap to maintain impedance <50kΩ, with a sampling rate of 500Hz, and performing offline filtering on the collected resting-state EEG data generated by methamphetamine-induced cues using a 0.01-100Hz bandpass filter or a 50Hz notch filter.

As a preferred embodiment of the present invention, step (2) specifically comprises the following steps:
(2.1) Performing offline preprocessing on the collected high-density EEG data, downsampling the time series to 250Hz, removing 50Hz power frequency noise through a notch filter, and performing bandpass filtering between 1Hz and 100Hz through a zero-phase finite impulse response filter;
(2.2) Removing bad channels including bridged channels and noisy channels and performing spherical interpolation, excluding and excising paroxysmal segments; using independent component analysis to decompose independent components, including activities originating from muscle motor unit groups (such as eyes) and strong highfrequency broadband activities that aggregate many action potentials during muscle contraction and static tension. Specifically, performing modal decomposition on the interpolated EEG signal: the time series recorded on the scalp is a spatially stable mixture of temporally independent brain and artifact activities. First, find a "decomposition" matrix W that decomposes or linearly decomposes the multi-channel scalp data into the sum of temporally independent and spatially fixed components. The rows of the output data matrix U = WX are the time courses of principal component activation. The columns of the inverse matrix inv(W) give the relative projection intensity of each component on each scalp sensor (right). These scalp weights give each component a scalp topography and provide evidence for the physiological origin of these components.
(2.3) Detecting artifact components using principal component analysis, obtaining the scalp topography of the four selected components and the artifact-corrected EEG time series obtained by removing the four selected electrooculographic and muscle noise components from the data;
(2.4) Filtering the sensor-level preprocessed time series into five typical frequency bands: delta (1-3Hz), theta (4-7Hz), alpha (8-12Hz), beta (13-30Hz) and gamma (31-50Hz) through a custom pipeline and verification by EEG experts, thereby completing data preprocessing.

As a preferred embodiment of the present invention, step (3) specifically comprises the following steps:
(3.1) Calculating a three-layer symmetric boundary element model of the head using OpenMEEG, then selecting rotating dipoles at 3003 vertices on the cortical surface, estimating an imaging kernel using a minimum norm estimation method with depth weighting and regularization, wherein the imaging kernel maps scalp electrode EEG to cortical source-level current source density, and then reducing the current density time series of each vertex in three spatial dimensions from three orthogonal axes to one dimension through principal component analysis;
(3.2) Generating a time series representing the main variation patterns of all vertices in the same region of interest through principal component analysis, extracting the dominant signal of the time series using singular value decomposition, and regarding the first singular vector as the signal at the region of interest level;
(3.3) The 31 regions of interest are independently divided according to the functional connectivity of resting-state functional magnetic resonance imaging, including VIS (Visual Network): Bilateral primary visual areas (V1); Somatosensory Network: Bilateral primary somatosensory cortex (S1); DAN (Dorsal Attention Network): Inferior frontal junction (IFJ), intraparietal sulcus (IPS), frontal eye field (FEF) and supplementary eye field (SEF); DMN (Default Mode Network): Posterior cingulate cortex (PCC), medial prefrontal cortex (MPFC) and angular gyrus (ANG); FPN (Frontoparietal Control Network): Posterior middle frontal gyrus (PMFG), inferior parietal lobule (IPL), orbital gyrus (ORB) and middle temporal gyrus (MTG); and VAN (Ventral Attention Network): Anterior middle frontal gyrus (AMFG), insular gyrus (INS), dorsal anterior cingulate cortex (DACC) and supramarginal gyrus (SUP).

As a preferred embodiment of the present invention, step (4) is specifically:
Calculating the imaginary coherence connectivity of 465 (31 × 31) pairs of regions of interest for each of the five frequency bands and each of the two resting eye conditions to obtain interactions between brain regions of interest at the cortical level. Let *S_{ij,t}*(*f*) denote the cross-spectral density of *x_{_i}*(*t*) and *x_{_j}*(*t*) at time point or trial t, where *S_{ij,t}*(*f*) is expressed as: ${S}_{ij , t} \left(f\right) = \left〈{x}_{i , t}\left(f\right){{x}_{j , t}}^{*}\left(f\right)\right〉 ,$ where * denotes complex conjugate, < > denotes expected value, *x_{_i}*(*t*) is the signal of brain region *i* at time point, and *x_{_j}*(*t*) is the signal of brain region *j* at time point.

Let *S_{_j,j}*(*f*) denote the cross-spectrum, and imaginary coherence is defined as: ${iCoh}_{ij} \left(f\right) = Imag \left({Coh}_{ij}\left(f\right)\right) = \frac{Imag \left({S}_{ij}\left(f\right)\right)}{\sqrt{{S}_{ii} \left(f\right) ⋅ {S}_{jj} \left(f\right)}} .$

Calculation method: The imaginary part of the electrode cross-spectrum divided by the square root of the power of each brain region, where *Sᵢⱼ*(*f*) is the cross-spectrum of brain region *i* and brain region *j*, and *Sᵢᵢ*(*f*) and *Sⱼⱼ* are the powers of each brain region, respectively.

As a preferred embodiment of the present invention, step (5) specifically comprises the following steps:
(5.1) Performing group-level analysis on the imaginary coherence of source-space functional connectivity, and correcting the p-value of each pair of functional connections using false discovery rate adjustment;
(5.2) Using biomarker-based machine learning models for craving prediction in addicted individuals and identification of abnormal addicted individuals to assess the strength of the relationship between the two biomarkers;
(5.3) Constructing a sparse linear regression model for predicting craving scores from connectivity features using a Relevance Vector Machine with a linear kernel for high-density EEG data, and establishing a sparse classification model to distinguish methamphetamine addicts from healthy individuals using an RVM with a linear kernel for high-density EEG data and a sigmoid function outer layer;
(5.4) Using the RVM machine learning model to penalize complex models by maximizing marginal likelihood with sparse priors, thereby automatically selecting relevant features for prediction;
(5.5) Craving prediction for methamphetamine addicts: Predicting craving scores in methamphetamine addicts based on brain functional connectivity in regions of interest using an RVM model with 10 repetitions of 10-fold cross-validation;
(5.6) Abnormality identification for methamphetamine addicts: Classifying methamphetamine addicts and healthy control individuals using an RVM model with 10 repetitions of 10-fold cross-validation based on high-density EEG functional connectivity estimates, wherein the model assigns label "1" to individuals with methamphetamine addiction and label "0" to healthy control individuals, and evaluating classification performance using classification accuracy, sensitivity, specificity and area under the receiver operating characteristic curve;
(5.7) Abnormality identification and craving prediction using source-level power: Using the power spectrum of each frequency band in a single region of interest space as input features for the machine learning model, and comparing the classification and prediction performance between electrode functional connectivity and frequency band power spectra. In practical applications, this technical solution aims to use high-density scalp EEG data for craving prediction and abnormality identification. It analyzes high-density scalp EEG data by calculating cortical functional connectivity after source localization to achieve the following two main goals: (1) predict the craving level of methamphetamine addicts, and (2) identify differences between methamphetamine addicts and healthy individuals.

Working steps and implementation technical details:
1. Data acquisition: Adjust all 128 scalp channels to maintain impedance <50kΩ. The sampling rate is 500Hz, and offline filtering is performed using a 0.01-100Hz bandpass filter (using a 50Hz notch filter). Participants are asked to record post-induction resting-state high-density EEG after watching a 5-minute video of methamphetamine-inducing cues: sitting with eyes closed in a quiet room for 5 minutes, staying awake and relaxed, then staring at a fixed point with eyes open for 5 minutes, and then closing eyes for another 5 minutes. Custom MATLAB scripts are used for preprocessing and source localization.
2. Preprocessing: Perform offline preprocessing on high-density EEG data, downsampling the time series to 250Hz, removing 50Hz power frequency noise by notch filtering, and performing bandpass filtering between 1Hz and 100Hz through a zero-phase finite impulse response filter. Then remove bad channels including bridged channels and noisy channels and perform spherical interpolation. Paroxysmal segments are excluded and excised. To remove artifacts such as blinks, eye movements, heartbeats and electromyography, independent component analysis (ICA) with an extended information maximization algorithm 59 is used to decompose independent components. Then principal component analysis (PCA) is used to detect and remove bad components through dimensionality reduction, and the minimum number of principal components accounting for more than 99.9% of the total variance is determined as the dimension. After the final re-reference, preprocessed EEG time series are obtained. Through a custom pipeline and EEG expert verification, extreme values of flat-line amplitude or outliers, channel correlations and robust paroxysmal spike measurements can be detected and eliminated by an automatic algorithm 60. The sensor-level preprocessed time series are filtered into five typical frequency bands: delta (1-3Hz), theta (4-7Hz), alpha (8-12Hz), beta (13-30Hz) and gamma (31-50Hz).
3. Source localization: Perform source localization based on a standard head model. Calculate a three-layer symmetric boundary element model of the head using OpenMEEG, then select rotating dipoles at 3003 vertices on the cortical surface. Estimate an imaging kernel using the minimum norm estimation (MNE) method with depth weighting and regularization, which maps scalp electrode EEG to cortical source-level current source density. Subsequently, reduce the current density time series of each vertex in three spatial dimensions from three orthogonal axes to one dimension through principal component analysis.
4. Connectivity analysis: Perform connectivity analysis in 31 regions of interest of the brain template defined by the Montreal Neurological Institute (MNI), which is derived from independent partitioning of functional connectivity of resting-state functional magnetic resonance imaging by independent component analysis clustering peaks. Generate time series representing the main variation patterns of all vertices in the same region of interest through principal component analysis. Extract the dominant signal of the time series using singular value decomposition (SVD), and regard the first singular vector as the signal at the region of interest level. These 31 regions of interest are divided into six brain functional networks: VIS (Visual Network): Bilateral primary visual areas (V1); Somatosensory Network: Bilateral primary somatosensory cortex (S1); DAN (Dorsal Attention Network): Inferior frontal junction (IFJ), intraparietal sulcus (IPS), frontal eye field (FEF) and supplementary eye field (SEF); DMN (Default Mode Network): Posterior cingulate cortex (PCC), medial prefrontal cortex (MPFC) and angular gyrus (ANG); FPN (Frontoparietal Control Network): Posterior middle frontal gyrus (PMFG), inferior parietal lobule (IPL), orbital gyrus (ORB) and middle temporal gyrus (MTG); and VAN (Ventral Attention Network): Anterior middle frontal gyrus (AMFG), insular gyrus (INS), dorsal anterior cingulate cortex (DACC) and supramarginal gyrus (SUP).
5. Functional connectivity network calculation: Functional connectivity networks (FCNs) play a crucial role in capturing dynamic interactions by detecting temporal correlations or phase synchronization between multiple brain regions. Coherence and coherency are two widely used methods for analyzing EEG connectivity. To reduce the impact of volume conduction of scalp signals, a solution involving extracting imaginary coherence, which represents the time delay between two channels, has been developed. In this study, imaginary coherence (iCoh) is used to reveal cortical interactions. Here, iCoh connectivity is calculated for 465 unique ROI pairs in each of the 5 frequency bands and each of the 2 resting eye conditions.

In this technical solution, we use imaginary coherence (iCoh) to reveal interactions between brain regions of interest at the cortical level. Calculation method: The imaginary part of the electrode cross-spectrum divided by the square root of the power of each brain region. Let *S(ij, t)* denote the cross-spectral density of *x_i*(*t*) and *x_j*(*t*) at time point or trial t, *S(ij, t)* is defined as: ${S}_{ij , t} \left(f\right) = \left〈{x}_{i , t}\left(f\right){{x}_{j , t}}^{*}\left(f\right)\right〉 ,$ where * denotes complex conjugate and < > denotes expected value.

Let S_ij(f) denote the cross-spectrum, and imaginary coherence is defined as: ${iCoh}_{ij} \left(f\right) = Imag \left({Coh}_{ij}\left(f\right)\right) = \frac{Imag \left({S}_{ij}\left(f\right)\right)}{\sqrt{{S}_{ii} \left(f\right) ⋅ {S}_{jj} \left(f\right)}} ,$

Due to the antisymmetry of the imaginary part, calculating this connectivity at the region of interest level by averaging the correlation of each source-space vertex between two given region of interest nodes may lead to errors [iCoh]_ij(f) =-[iCoh]_ji(f)). Therefore, to avoid this error, iCoh at the region of interest level in the carrier frequency band is directly calculated.

### 6. Group analysis:

(1) Perform group-level analysis on source-space FCN (functional connectivity, imaginary coherence iCoh). To investigate the relationship between craving and FCN, we calculate the correlation between these two factors while correcting p-values for multiple comparisons using false discovery rate (FDR) adjustment. In addition, we perform an independent t-test to compare FCN between the methamphetamine addiction group and the healthy control group, correcting the p-value of each pair of functional connections using FDR adjustment. Furthermore, we calculate the correlation between craving and behavioral measures while correcting with FDR adjustment.
(2) Machine learning models: Two machine learning models based on two biomarkers (craving prediction in addicted individuals and identification of abnormal addicted individuals) are used to evaluate the strength of this relationship. For both models, brain functional connectivity measures between each region of interest are used. Please refer to the figure for the machine learning workflow.
(3) Relevance Vector Machine: A Relevance Vector Machine (RVM) with a linear kernel for high-density EEG data is used to construct a sparse linear regression model for predicting craving scores from connectivity features. An RVM with a linear kernel for high-density EEG data and a sigmoid function outer layer is also used to establish a sparse classification model to distinguish methamphetamine addicts from healthy individuals.
(4) RVM is a machine learning technique that automatically selects relevant features for prediction by penalizing complex models through maximizing marginal likelihood with sparse priors in a sparse Bayesian learning framework. Compared with Support Vector Machines (SVMs), RVM can provide probabilistic predictions, reduce computational complexity, and estimate error/margin trade-off parameters. In addition, the kernel function of RVM does not need to satisfy Mercer's condition, which means it can determine hyperparameters without additional validation.
(5) Craving prediction in methamphetamine addicts: Use an RVM model with 10 repetitions of 10-fold cross-validation (data are randomly divided into 10 subsets, each containing approximately the same number of subjects. One subset is selected as test data, and the other 9 subsets are used as training data. This process is repeated 10 times, with each of the 10 subsets used once as test data. Therefore, each subject has a predicted probability.

To improve prediction stability, the data are randomly grouped 10 times, and stratified 10-fold cross-validation is performed on each random dataset) to predict craving scores in methamphetamine addicts based on brain functional connectivity in regions of interest. We evaluate prediction performance by calculating the Pearson correlation coefficient and root mean square error (RMSE) between cross-validated predicted scores and true scores. We also report the p-value of the one-tailed alternative hypothesis that the coefficient of determination of iCoh in the five bands is greater than 0 (a total of 10 patterns) under resting eyes-open and eyes-closed conditions after correction for multiple comparisons using the FDR method. In addition, since the RVM model is a sparse model, it has the ability to use the feature weight matrix of the RVM model to indicate the predictive importance and capability of brain functional connectivity.

(6) Abnormality identification in methamphetamine addicts: Based on high-density EEG functional connectivity estimates, classify methamphetamine addicts and healthy control individuals using an RVM model with 10 repetitions of 10-fold cross-validation. The model assigns label "1" to individuals with methamphetamine addiction and label "0" to healthy control individuals. Classification performance is evaluated using classification accuracy, sensitivity (true positive rate), specificity (true negative rate) and area under the receiver operating characteristic curve (AUC). Higher AUC values indicate better separability and higher classification performance. The diagnostic odds ratio, defined as the ratio of the odds of testing positive when the subject has the disease to the odds of testing positive when the subject does not have the disease, is also calculated to measure the effectiveness of the classification model.

(7) Abnormality identification and craving prediction using sensor-space power: To test the predictive ability of brain functional connectivity in methamphetamine addicts, the power spectrum of each frequency band in a single region of interest space is also used as input features for the machine learning model, and the classification and prediction performance between cortical functional connectivity and frequency band power spectra are compared.

In practical applications, this technical solution has the following significant technical effects:
1. EEG feature machine learning model predicts methamphetamine craving: Models based on REOβ and REOδ can significantly predict craving scores of methamphetamine addicts. Specifically, the REOδ model has a Pearson's r of 0.41, p=0.009 (FDR corrected), and a root mean square error of 21.60, while the REOβ model shows the highest performance with Pearson's r=0.73, p<3×10⁻⁹ (FDR corrected), and root mean square error=14.75.
2. EEG feature machine learning model identifies methamphetamine addicts: Evaluated by 10 repetitions of 10-fold cross-validation. The REOβ model shows the best classification performance across all frequency bands and resting conditions, achieving a classification accuracy of 80.95% (sensitivity: 82.14%; specificity: 79.59%), which is significantly higher than other models (all models have classification accuracy below 70%). In addition, compared with other frequency bands and resting conditions, the REOβ model also shows the highest AUC (0.859) and diagnostic odds ratio (DOR, 17.94).

We tested whether the craving prediction model could be replicated by performing the same analysis on an independent sample of methamphetamine addicts' induced-state resting-state EEG dataset. First, we tested the model performance of the best predictor REOβ trained on Dataset 1 using Dataset 2, and observed a relatively high performance level (Pearson's r=0.60, p<2×10⁻⁵, RMSE=19.31), only slightly lower than the performance on the training dataset. This result demonstrates the robustness of the craving prediction model trained on REOβ of the previous dataset. Next, we used Methamphetamine Addiction Dataset 2 as an independent dataset and performed 10 repetitions of 10-fold cross-validation. The prediction performance of models established for all frequency bands and resting conditions indicates that the REOβ model can significantly predict craving scores (Pearson's r=0.65, p<2×10⁻⁵, FDR corrected, RMSE=17.56), consistent with the results found in the Methamphetamine Addicts Dataset. However, compared with the previous dataset, the REOδ model did not show significant prediction (Pearson's r=0.29, p=0.222, FDR corrected, RMSE=22.78). Consistent with the results obtained on the discovery dataset, the results of the REOβ model trained on Methamphetamine Addicts Dataset 2 demonstrate the robustness of the trained RVM model in predicting craving in methamphetamine addicts. Finally, we evaluated the weights of the RVM model for REOβ trained on Dataset 2. As with the weights trained on Dataset 1, we observed that connections between the medial prefrontal cortex and the right supramarginal gyrus (e.g., prefrontal cortex-right supramarginal gyrus) play an important role in prediction.

Individual-level biomarkers: The invention provides a biomarker capable of measuring craving at the individual level through machine learning algorithms, which is of great significance in the diagnosis and treatment of methamphetamine addiction because the degree of craving may vary among each patient.

Application of scalp EEG data: This technical solution innovatively utilizes high-density scalp EEG data and combines it with craving-related brain functional connectivity networks to achieve more accurate diagnosis and monitoring.

Clinical pathway for personalized treatment: This technical solution provides a new approach for individual-level treatment methods, which is expected to improve the treatment effect of methamphetamine addicts and reduce the relapse rate.

The apparatus for realizing methamphetamine addiction identification and craving determination based on machine learning comprises:
A processor configured to execute computer-executable instructions;
A memory storing one or more computer-executable instructions, which when executed by the processor, implement the steps of the above-described method for realizing methamphetamine addiction identification and craving determination based on machine learning.

The processor for realizing methamphetamine addiction identification and craving determination based on machine learning is configured to execute computer-executable instructions, which when executed by the processor, implement the steps of the above-described method for realizing methamphetamine addiction identification and craving determination based on machine learning.

The computer-readable storage medium has a computer program stored thereon, which can be executed by a processor to implement the steps of the above-described method for realizing methamphetamine addiction identification and craving determination based on machine learning.

Any process or method description in the flowcharts or otherwise described herein may be understood as representing a module, segment or portion of code comprising one or more executable instructions for implementing specific logical functions or steps of the process, and the scope of preferred embodiments of the present invention includes alternative implementations in which functions may be performed out of the order shown or discussed, including substantially concurrently or in reverse order depending on the functionality involved, as should be understood by those skilled in the art to which the embodiments of the present invention pertain.

It should be understood that each part of the present invention can be implemented by hardware, software, firmware or a combination thereof. In the above embodiments, multiple steps or methods may be implemented by software or firmware stored in a memory and executed by a suitable instruction execution device.

Those of ordinary skill in the art will appreciate that all or part of the steps carried by the method of implementing the above embodiments may be completed by a program instructing relevant hardware. The program may be stored in a computer-readable storage medium, which when executed, includes one or a combination of the steps of the method embodiments.

The above-mentioned storage medium may be a read-only memory, a magnetic disk, an optical disk, or the like.

In the description of this specification, reference to the terms "one embodiment", "some embodiments", "example", "specific example", or "embodiment" means that a particular feature, structure, material, or characteristic described in connection with the embodiment or example is included in at least one embodiment or example of the present invention. In this specification, the schematic representations of the above terms do not necessarily refer to the same embodiment or example. Moreover, the particular features, structures, materials, or characteristics described may be combined in any suitable manner in any one or more embodiments or examples.

Although the embodiments of the present invention have been shown and described above, it is to be understood that the above embodiments are exemplary and are not to be construed as limiting the present invention, and that those skilled in the art may make changes, modifications, substitutions and variations to the above embodiments within the scope of the present invention.

By adopting the method, apparatus, processor and computer-readable storage medium for realizing methamphetamine addiction identification and craving determination based on machine learning of the present invention, major innovations have been brought to the diagnosis and treatment of methamphetamine addiction by combining high-density scalp EEG data and analysis of neural connection networks, providing a new approach for individual-level treatment methods, which is expected to improve the treatment effect of methamphetamine addicts and reduce the relapse rate.

In this specification, the present invention has been described with reference to specific embodiments thereof. However, it is apparent that various modifications and changes may be made thereto without departing from the spirit and scope of the present invention. Accordingly, the specification and drawings are to be regarded in an illustrative rather than a restrictive sense.

## Claims

1. A method for realizing methamphetamine addiction identification and craving determination based on machine learning, **characterized in that** the method comprises the following steps:
(1) Collecting high-density scalp resting-state EEG data from subjects;
(2) Preprocessing the collected data to remove noise data and artifacts, obtaining preprocessed EEG time series;
(3) Performing brain region source localization processing on the acquired time series data using a predefined brain template based on a standard head model;
(4) Detecting temporal correlations or phase synchronization between multiple brain regions using a functional connectivity network;
(5) Performing feature discrimination of the subjects' true craving performance by establishing and validating a machine learning model.

2. The method for realizing methamphetamine addiction identification and craving determination based on machine learning according to claim 1, **characterized in that** step (1) is specifically:
Adjusting all 128 scalp channels of the high-density EEG cap to maintain impedance <50kΩ, with a sampling rate of 500Hz, and performing offline filtering on the collected resting-state EEG data generated by methamphetamine-induced cues using a 0.01-100Hz bandpass filter or a 50Hz notch filter.

3. The method for realizing methamphetamine addiction identification and craving determination based on machine learning according to claim 1, **characterized in that** step (2) specifically comprises the following steps:
(2.1) Performing offline preprocessing on the collected high-density EEG data, downsampling the time series to 250Hz, removing 50Hz power frequency noise through a notch filter, and performing bandpass filtering between 1Hz and 100Hz through a zero-phase finite impulse response filter;
(2.2) Removing bad channels including bridged channels and noisy channels and performing spherical interpolation, excluding and excising paroxysmal segments; decomposing independent components using independent component analysis to remove artifacts such as blinks, eye movements, heartbeats and electromyography;
(2.3) Detecting and removing bad components through dimensionality reduction using principal component analysis, and determining the minimum number of principal components accounting for more than 99.9% of the total variance as the dimension, thereby obtaining preprocessed EEG time series;
(2.4) Filtering the sensor-level preprocessed time series into five typical frequency bands: delta (1-3Hz), theta (4-7Hz), alpha (8-12Hz), beta (13-30Hz) and gamma (31-50Hz) through a custom pipeline and verification by EEG experts, thereby completing data preprocessing.

4. The method for realizing methamphetamine addiction identification and craving determination based on machine learning according to claim 3, **characterized in that** step (3) specifically comprises the following steps:
(3.1) Calculating a three-layer symmetric boundary element model of the head using OpenMEEG, then selecting rotating dipoles at 3003 vertices on the cortical surface, estimating an imaging kernel using a minimum norm estimation method with depth weighting and regularization, wherein the imaging kernel maps scalp electrode EEG to cortical source-level current source density, and then reducing the current density time series of each vertex in three spatial dimensions from three orthogonal axes to one dimension through principal component analysis;
(3.2) Generating a time series representing the main variation patterns of all vertices in the same region of interest through principal component analysis, extracting the dominant signal of the time series using singular value decomposition, and regarding the first singular vector as the signal at the region of interest level;
(3.3) The 31 regions of interest are independently divided according to the functional connectivity of resting-state functional magnetic resonance imaging, including VIS (Visual Network): Bilateral primary visual areas (V1); Somatosensory Network: Bilateral primary somatosensory cortex (S1); DAN (Dorsal Attention Network): Inferior frontal junction (IFJ), intraparietal sulcus (IPS), frontal eye field (FEF) and supplementary eye field (SEF); DMN (Default Mode Network): Posterior cingulate cortex (PCC), medial prefrontal cortex (MPFC) and angular gyrus (ANG); FPN (Frontoparietal Control Network): Posterior middle frontal gyrus (PMFG), inferior parietal lobule (IPL), orbital gyrus (ORB) and middle temporal gyrus (MTG); and VAN (Ventral Attention Network): Anterior middle frontal gyrus (AMFG), insular gyrus (INS), dorsal anterior cingulate cortex (DACC) and supramarginal gyrus (SUP).

5. The method for realizing methamphetamine addiction identification and craving determination based on machine learning according to claim 4, **characterized in that** step (4) is specifically:
Calculating the imaginary coherence connectivity of 465 unique ROI pairs for each of the five frequency bands and each of the two resting eye conditions to obtain interactions between brain regions of interest at the cortical level. Let *S_{ij,t}*(*f*) denote the cross-spectral density of *x_{_i}*(*t*) and *x_{_j}*(*t*) at time point or trial *t,* where *S_{ij,t}*(*f*) is expressed as: ${S}_{ij , t} \left(f\right) = \left〈{x}_{i , t}\left(f\right){{x}_{j , t}}^{*}\left(f\right)\right〉 ,$
where * denotes complex conjugate and < > denotes expected value;
Let *Sᵢⱼ*(*f*) denote the cross-spectrum, and imaginary coherence is defined as: ${iCoh}_{ij} \left(f\right) = Imag \left({Coh}_{ij}\left(f\right)\right) = \frac{Imag \left({S}_{ij}\left(f\right)\right)}{\sqrt{{S}_{ii} \left(f\right) ⋅ {S}_{jj} \left(f\right)}}$
where *Sᵢⱼ*(*f*) is the cross-spectrum of brain region *i* and brain region *j*, and *Sᵢᵢ*(*f*) and *Sⱼⱼ* are the powers of each brain region, respectively.

6. The method for realizing methamphetamine addiction identification and craving determination based on machine learning according to claim 5, **characterized in that** step (5) specifically comprises the following steps:
(5.1) Performing group-level analysis on source-space FCNs and correcting the p-value of each pair of functional connections using false discovery rate adjustment;
(5.2) Using biomarker-based machine learning models for craving prediction in addicted individuals and identification of abnormal addicted individuals to assess the strength of the relationship between the two biomarkers;
(5.3) Constructing a sparse linear regression model for predicting craving scores from connectivity features using a Relevance Vector Machine with a linear kernel for high-density EEG data, and establishing a sparse classification model to distinguish methamphetamine addicts from healthy individuals using an RVM with a linear kernel for high-density EEG data and a sigmoid function outer layer;
(5.4) Using the RVM machine learning model to penalize complex models by maximizing marginal likelihood with sparse priors, thereby automatically selecting relevant features for prediction;
(5.5) Craving prediction for methamphetamine addicts: Predicting craving scores in methamphetamine addicts based on brain functional connectivity in regions of interest using an RVM model with 10 repetitions of 10-fold cross-validation;
(5.6) Abnormality identification for methamphetamine addicts: Classifying methamphetamine addicts and healthy control individuals using an RVM model with 10 repetitions of 10-fold cross-validation based on high-density EEG functional connectivity estimates, wherein the model assigns label "1" to individuals with methamphetamine addiction and label "0" to healthy control individuals, and evaluating classification performance using classification accuracy, sensitivity, specificity and area under the receiver operating characteristic curve;
(5.7) Abnormality identification and craving prediction using sensor-space power: Using the power spectrum of each frequency band in a single region of interest space as input features for the machine learning model, and comparing the classification and prediction performance between cortical functional connectivity and frequency band power spectra.

7. An apparatus for realizing methamphetamine addiction identification and craving determination based on machine learning, **characterized in that** the apparatus comprises:
A processor configured to execute computer-executable instructions;
A memory storing one or more computer-executable instructions, which when executed by the processor, implement the steps of the method for realizing methamphetamine addiction identification and craving determination based on machine learning according to any one of claims 1 to 6.

8. A processor for realizing methamphetamine addiction identification and craving determination based on machine learning, **characterized in that** the processor is configured to execute computer-executable instructions, which when executed by the processor, implement the steps of the method for realizing methamphetamine addiction identification and craving determination based on machine learning according to any one of claims 1 to 6.

9. A computer-readable storage medium, **characterized in that** a computer program is stored thereon, which can be executed by a processor to implement the steps of the method for realizing methamphetamine addiction identification and craving determination based on machine learning according to any one of claims 1 to 6.
